# EUROPEAN PATENT APPLICATION

(11) **EP 3 750 484 A1**
(43) Date of publication of application: **16.12.2020**
(21) Application number: 20179566.3
(22) Date of filing: 11.06.2020
(51) Int. Cl.: A61B 8/08, A61B 8/00

(54) **ATTACHMENT AND ULTRASOUND PROBE**

(30) Priority: 13.06.2019 JP 2019110466
(71) Applicant: FUJIFILM Corporation, Tokyo 106-8620 (JP)
(72) Inventor: FUJIMOTO, Yoshie, Ashigarakami-gun, Kanagawa 258-8538 (JP); NAKAYAMA, Hiroki, Ashigarakami-gun, Kanagawa 258-8538 (JP); ARAI, Takahisa, Ashigarakami-gun, Kanagawa 258-8538 (JP); KODAIRA, Shunsuke, Ashigarakami-gun, Kanagawa 258-8538 (JP)
(74) Representative: Jeffrey, Philip Michael

(57) **Abstract**

Provided are an attachment and an ultrasound probe which can improve the convenience of providing an acoustic matching member.

The attachment includes: a holder that has an opening portion provided at a position corresponding to an ultrasonic wave transmitting and receiving surface of the ultrasound probe and is attachably and detachably fixed to the ultrasound probe; and a protruding portion that draws and holds a sheet-shaped acoustic matching member provided between the opening portion and the ultrasound probe in a close contact direction in which the acoustic matching member is brought into close contact with the transmitting and receiving surface.

## Description

### BACKGROUND OF THE INVENTION

### 1. Field of the Invention

The present disclosure relates to an attachment and an ultrasound probe.

### 2. Description of the Related Art

An ultrasonography apparatus has been known which captures an ultrasound image of a subject using an ultrasound probe. Generally, in a case in which an ultrasound image is captured, an acoustic matching member is provided between an ultrasound probe and a subject in order to reduce a mismatch in acoustic impedance.

As a technique for providing an acoustic matching member in an ultrasound probe, for example, JP2015-205124A discloses a technique that attaches a housing for accommodating an acoustic matching member to an ultrasound probe to ensure acoustic matching between the ultrasound probe and a subject.

### SUMMARY OF THE INVENTION

However, it is known that it takes a lot of time and effort for a user to provide the acoustic matching member between the ultrasound probe and the subject. Therefore, it is desirable to improve the convenience of the user for providing the acoustic matching member. However, in the technique disclosed in JP2015-205124A, in some cases, the convenience of the user is not sufficient.

The present disclosure has been made in view of the above-mentioned problems and an object of the present disclosure is to provide an attachment and an ultrasound probe that can improve the convenience of providing an acoustic matching member.

In order to achieve the above object, according to a first aspect of the present disclosure, there is provided an attachment for an ultrasound probe. The attachment comprises: a holder that has an opening portion provided at a position corresponding to an ultrasonic wave transmitting and receiving surface of the ultrasound probe and is attachably and detachably fixed to the ultrasound probe; and a protruding portion that draws and holds a sheet-shaped acoustic matching member provided between the opening portion and the ultrasound probe in a close contact direction in which the acoustic matching member is brought into close contact with the transmitting and receiving surface.

According to a second aspect of the present disclosure, in the attachment according to the first aspect, the protruding portion may be provided on a surface of the holder which faces the ultrasound probe.

According to a third aspect of the present disclosure, in the attachment according to the first aspect, the holder may have a first holder that is attachably and detachably fixed to the ultrasound probe and a second holder that is attached to the first holder and has the opening portion. The protruding portion is provided on a surface of the second holder which faces the ultrasound probe.

According to a fourth aspect of the present disclosure, in the attachment according to the third aspect, the second holder may be attached to the first holder by being fastened to the first holder with a fastening member.

According to a fifth aspect of the present disclosure, in the attachment according to the fourth aspect, the protruding portion may draw the acoustic matching member in the close contact direction by fastening the second holder to the first holder with the fastening member.

According to a sixth aspect of the present disclosure, in the attachment according to any one of the second to fifth aspects, an angle formed between a tip of the protruding portion and a contact surface of the acoustic matching member with the tip may be equal to or greater than 70 degrees and equal to or less than 120 degrees.

According to a seventh aspect of the present disclosure, in the attachment according to any one of the first to sixth aspects, the acoustic matching member may protrude from an opening end of the holder in a state in which the attachment is attached to the ultrasound probe.

According to an eighth aspect of the present disclosure, in the attachment according to any one of the first to seventh aspects, a tip of the transmitting and receiving surface of the ultrasound probe may protrude from the opening end of the holder in a state in which the attachment is attached to the ultrasound probe.

According to a ninth aspect of the present disclosure, in the attachment according to the first aspect, the acoustic matching member may include an elastic attachment member which is provided on each of a pair of opposite sides. The protruding portion may be provided on a surface of the holder which is opposite to a surface facing the ultrasound probe. The attachment member may be attached to the protruding portion to draw the acoustic matching member in the close contact direction.

According to a tenth aspect of the present disclosure, in the attachment according to any one of the first to ninth aspects, a surface of the ultrasound probe may have at least one of a convex portion or a concave portion and the holder may have a shape that is fitted to the at least one.

In order to achieve the above object, according to an eleventh aspect of the present disclosure, there is provided an ultrasound probe to which the attachment according to any one of the first to tenth aspects is attached.

According to the present disclosure, it is possible to improve the convenience of providing an acoustic matching member.

### BRIEF DESCRIPTION OF THE DRAWINGS

Fig. 1 is a diagram schematically illustrating an example of the overall configuration of a medical imaging system according to a first embodiment.
Fig. 2 is a side view illustrating an example of the outward appearance of a mammography apparatus according to the first embodiment.
Fig. 3 is a block diagram illustrating an example of the configuration of an ultrasonography apparatus according to the first embodiment.
Fig. 4A is a front view illustrating an example of the outward appearance of an ultrasound probe according to the first embodiment.
Fig. 4B is a side view illustrating an example of the outward appearance of the ultrasound probe according to the first embodiment.
Fig. 5A is a front view illustrating an example of the outward appearance of an attachment according to the first embodiment.
Fig. 5B is a side view illustrating an example of the outward appearance of the attachment according to the first embodiment.
Fig. 6A is a front view illustrating an example of the outward appearance of the ultrasound probe in a state in which the attachment according to the first embodiment is attached.
Fig. 6B is a side view illustrating an example of the outward appearance of the ultrasound probe in a state in which the attachment according to the first embodiment is attached.
Fig. 6C is a cross-sectional view taken along line A-A of Fig. 6A.
Fig. 6D is a cross-sectional view taken along line B-B of Fig. 6A.
Fig. 7 is a cross-sectional view illustrating a mechanism in which an acoustic matching member is brought into close contact with a transmitting and receiving surface of the ultrasound probe by the attachment according to the first embodiment.
Fig. 8 is a cross-sectional view illustrating an example of the relationship between a protruding portion of a second holder and the acoustic matching member.
Fig. 9 is a cross-sectional view illustrating an example of a holder and an acoustic matching member in a state in which an attachment according to Modification Example 1 is attached.
Fig. 10A is a cross-sectional view illustrating an example of a holder and an acoustic matching member in a state in which an attachment according to Modification Example 2 is attached.
Fig. 10B is a cross-sectional view illustrating an example of a holder and an acoustic matching member in a state in which an attachment according to Modification Example 3 is attached.
Fig. 11A is a front view illustrating an example of the outward appearance of the ultrasound probe in a state in which an attachment according to a second embodiment is attached.
Fig.11B is a cross-sectional view taken along the line C-C of Fig. 11A.
Fig. 12A is a front view illustrating an example of the outward appearance of the ultrasound probe in a state in which an attachment according to a third embodiment is attached.
Fig. 12B is a side view illustrating an example of the outward appearance of the ultrasound probe in a state in which the attachment according to the third embodiment is attached.
Fig. 13 is a plan view illustrating an example of an acoustic matching member according to the third embodiment.

### DESCRIPTION OF THE PREFERRED EMBODIMENTS

Hereinafter, embodiments of the invention will be described in detail with reference to the drawings. Each of the embodiments does not limit the invention.

### First Embodiment

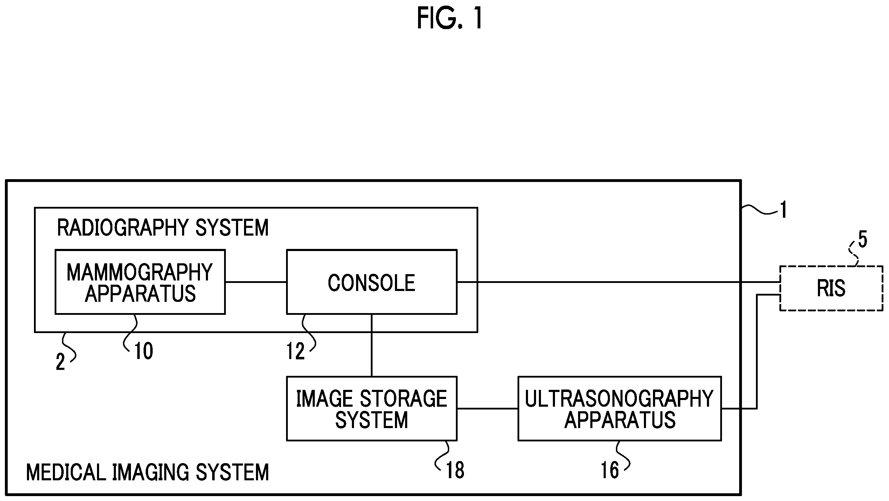

First, an example of the overall configuration of a medical imaging system according to this embodiment will be described. Fig. 1 is a diagram illustrating an example of the overall configuration of a medical imaging system 1 according to this embodiment.

As illustrated in Fig. 1, the medical imaging system 1 according to this embodiment comprises a radiography system 2, an ultrasonography apparatus 16, and an image storage system 18.

The image storage system 18 stores image data of a radiographic image captured by the radiography system 2 and image data of an ultrasound image captured by the ultrasonography apparatus 16. The image storage system 18 extracts an image corresponding to a request from, for example, the console 12, the ultrasonography apparatus 16, and other reading devices (not illustrated) from the stored radiographic images and ultrasound images and transmits the extracted image to the apparatus which is the request source. A specific example of the image storage system 18 is a picture archiving and communication system (PACS).

The radiography system 2 according to this embodiment includes a mammography apparatus 10 and a console 12. The console 12 according to this embodiment has a function of controlling the mammography apparatus 10 using, for example, an imaging order and various kinds of information acquired from a radiology information system (RIS) 5 through a wireless communication local area network (LAN) and commands input by the user through an operation unit (not illustrated) provided in the console 12. The console 12 according to this embodiment is, for example, a server computer comprising a central processing unit (CPU), a read only memory (ROM), a random access memory (RAM), and a storage unit.

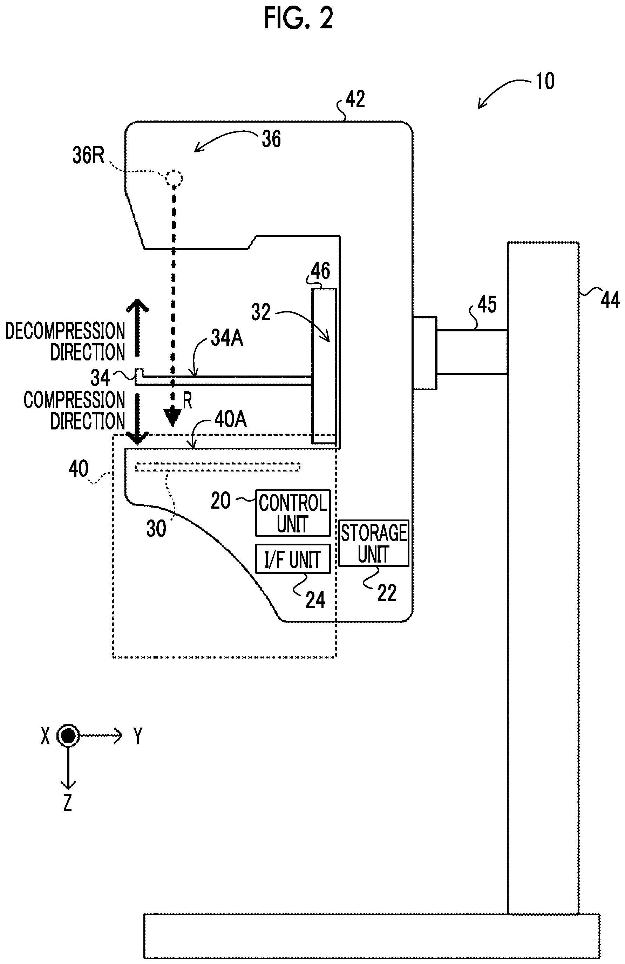

The mammography apparatus 10 according to this embodiment irradiates the breast of a subject as an object with radiation R (for example, X-rays) to capture a radiographic image of the breast. Fig. 2 is a side view illustrating an example of the outward appearance of the mammography apparatus 10 according to this embodiment. Fig. 2 illustrates an example of the outward appearance of the mammography apparatus 10 as viewed from the right side of the subject.

As illustrated in Fig. 2, the mammography apparatus 10 according to this embodiment comprises a control unit 20, a storage unit 22, and an interface (I/F) unit 24 which are provided in an imaging table 40. The control unit 20 controls the overall operation of the mammography apparatus 10 under the control of the console 12. The control unit 20 comprises a CPU, a ROM, and a RAM which are not illustrated. For example, various programs including an imaging processing program which is executed by the CPU and performs control related to the capture of a radiographic image are stored in the ROM in advance. The RAM temporarily stores various kinds of data.

For example, the image data of the radiographic image captured by the radiation detector 30 and various other kinds of information are stored in the storage unit 22. Examples of the storage unit 22 include a hard disk drive (HDD) and a solid state drive (SSD). The I/F unit 24 transmits and receives various kinds of information to and from the console 12 using wireless communication or wired communication. The image data of the radiographic image captured by the radiation detector 30 in the mammography apparatus 10 is transmitted to the console 12 through the I/F unit 24 by wireless communication or wired communication.

The radiation detector 30 detects the radiation R transmitted through the breast which is the object. As illustrated in Fig. 2, the radiation detector 30 is provided in the imaging table 40. In the mammography apparatus 10 according to this embodiment, in a case in which imaging is performed, the breast of the subject is positioned on an imaging surface 40A of the imaging table 40 by a user such as a doctor or a radiology technician.

The radiation detector 30 detects the radiation R transmitted through the breast of the subject and the imaging table 40, generates a radiographic image on the basis of the detected radiation R, and outputs image data indicating the generated radiographic image. The type of the radiation detector 30 according to this embodiment is not particularly limited. For example, the radiation detector 30 may be an indirect-conversion-type radiation detector that converts the radiation R into light and converts the converted light into charge or a direct-conversion-type radiation detector that directly converts the radiation R into charge.

The radiation emitting unit 36 comprises a radiation source 36R. As illustrated in Fig. 2, the radiation emitting unit 36 is provided in an arm portion 42 together with the imaging table 40 and a compression unit 46. The arm portion 42 is supported by a base 44 so as to be movable in the up-down direction (Z-axis direction). The shaft portion 45 connects the arm portion 42 to the base 44. The arm portion 42 can be relatively rotated with respect to the base 44, using a shaft portion 45 as a rotation axis.

The compression unit 46 and the arm portion 42 can be relatively rotated with respect to the base 44 separately, using the shaft portion 45 as a rotation axis. One or both of the arm portion 42 and the compression unit 46 connected to the shaft portion 45 are rotated integrally with the shaft portion 45.

The compression unit 46 is provided with a compression plate driving unit 32. The compression plate 34 according to this embodiment is a plate-shaped compression member and is moved in the up-down direction (Z-axis direction) by the compression plate driving unit 32 to compress the breast of the subject against the imaging table 40. As illustrated in Fig. 2, for the movement direction of the compression plate 34, the direction in which the breast is compressed, that is, the direction in which the compression plate 34 becomes closer to the imaging surface 40A is referred to as a "compression direction" and the direction in which the compression of the breast is released, that is, the direction in which the compression plate 34 becomes closer to the radiation emitting unit 36 is referred to as a "decompression direction".

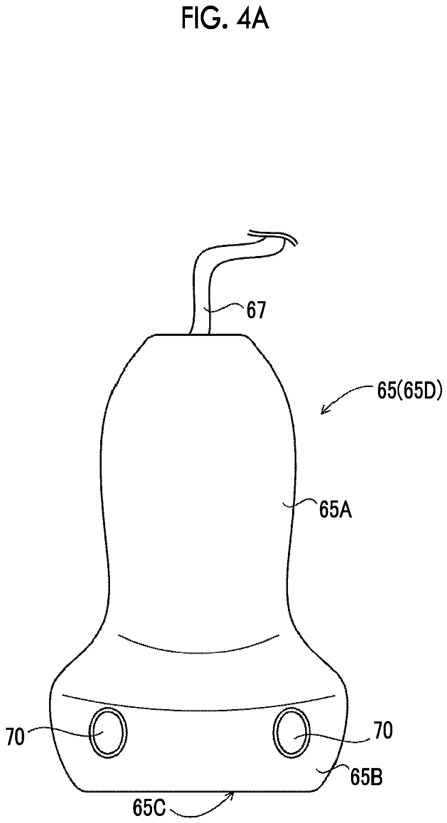

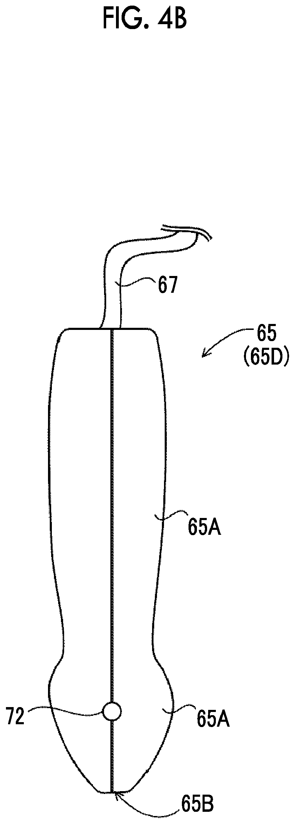

It is preferable that the compression plate 34 is optically transparent in order to check positioning or a compressed state in the compression of the breast. In addition, the compression plate 34 is made of a material having high transmittance for the radiation R. It is desirable that the compression plate 34 is made of a material that facilitates the transmission of ultrasonic waves from an ultrasound probe 65 (see Figs. 4A and 4B, which will be described in detail below) of the ultrasonography apparatus 16. Examples of the material forming the compression plate 34 include resins such as polymethylpentene, polycarbonate, acrylic, and polyethylene terephthalate. In particular, polymethylpentene is suitable as the material forming the compression plate 34 since it has low rigidity, high elasticity, and high flexibility and has suitable values for acoustic impedance that affects the reflectance of ultrasonic waves and an attenuation coefficient that affects the attenuation of ultrasonic waves. The member forming the compression plate 34 is not limited to this embodiment. For example, the member forming the compression plate 34 may be a film-like member.

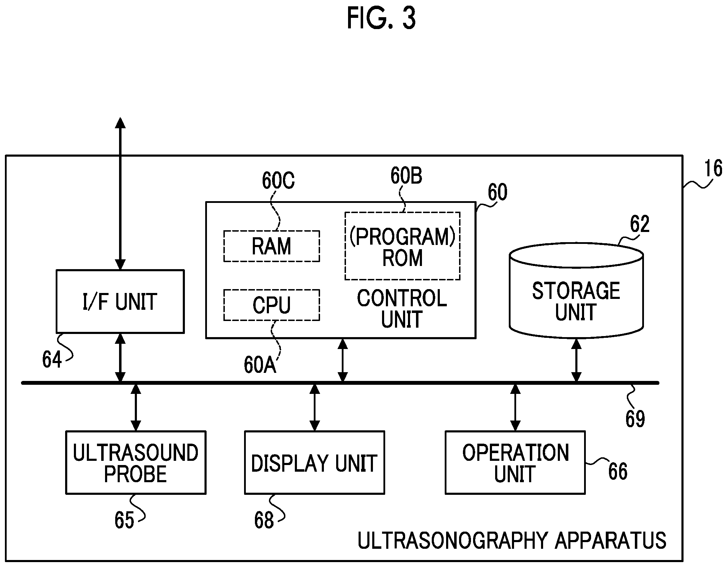

Next, the configuration of the ultrasonography apparatus 16 will be described. Fig. 3 is a block diagram illustrating an example of the configuration of the ultrasonography apparatus 16. The ultrasonography apparatus 16 is used by the user to capture an ultrasound image of the breast of the subject as the object and is a so-called hand-held ultrasonography apparatus.

As illustrated in Fig. 3, the ultrasonography apparatus 16 comprises a control unit 60, a storage unit 62, an I/F unit 64, the ultrasound probe 65, an operation unit 66, and a display unit 68. The control unit 60, the storage unit 62, the I/F unit 64, the ultrasound probe 65, the operation unit 66, and the display unit 68 are connected to each other through a bus 69, such as a system bus or a control bus, such that they can transmit and receive various kinds of information.

The control unit 60 according to this embodiment controls the overall operation of the ultrasonography apparatus 16. The control unit 60 comprises a CPU 60A, a ROM 60B, and a RAM 60C. For example, various programs executed by the CPU 60A are stored in the ROM 60B in advance. The RAM 60C temporarily stores various kinds of data.

For example, the image data of the captured ultrasound image and various other kinds of information are stored in the storage unit 62. A specific example of the storage unit 62 is an HDD or an SSD.

The operation unit 66 is used by the user to input, for example, commands or various kinds of information related to the capture of an ultrasound image. The operation unit 66 is not particularly limited. Examples of the operation unit 66 include various switches, a touch panel, a touch pen, and a mouse. The display unit 68 displays, for example, various kinds of information or an ultrasound image corresponding to the received signal from the ultrasound probe 65. In addition, the operation unit 66 and the display unit 68 may be integrated into a touch panel display.

The I/F unit 64 transmits and receives various kinds of information to and from the RIS 5 and the image storage system 18 using wireless communication or wired communication. The image data of the ultrasound image captured by the ultrasonography apparatus 16 is transmitted to the image storage system 18 through the I/F unit 64 by wireless communication or wired communication.

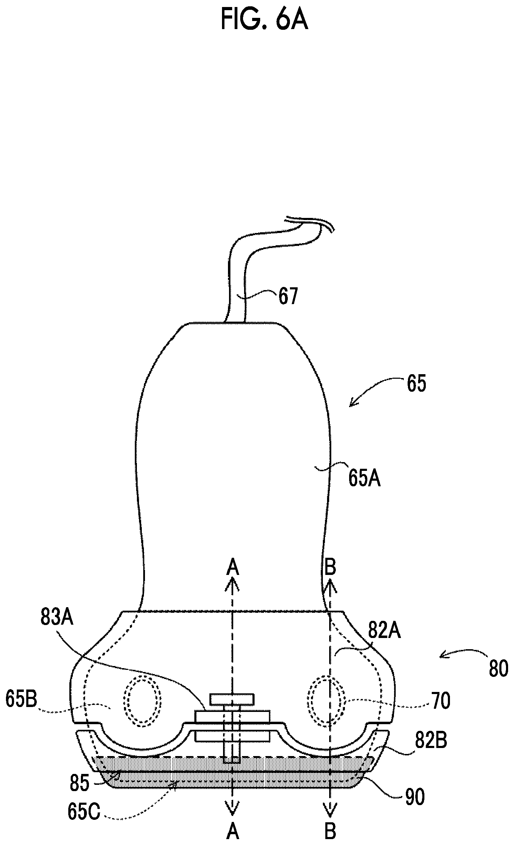

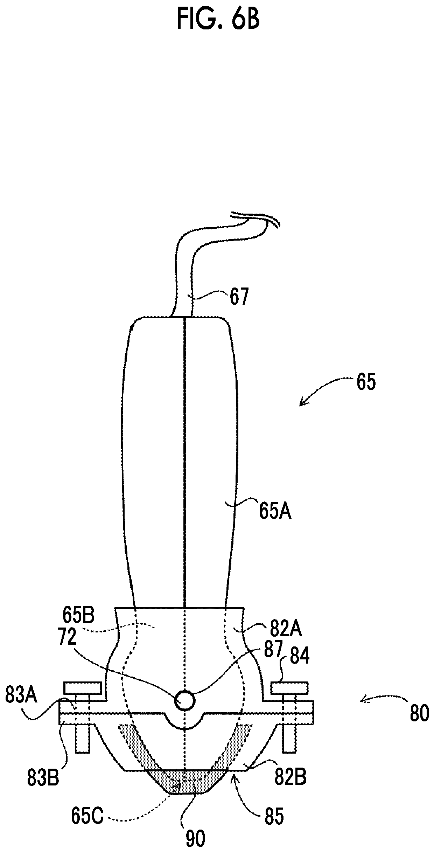

The ultrasound probe 65 is moved along the upper surface 34A (see Fig. 2, a surface opposite to the surface that comes into contact with the breast of the subject) of the compression plate 34 by the user and scans the breast with ultrasonic waves to acquire an ultrasound image of the breast. Specifically, in a case in which ultrasonography is performed, the ultrasound probe 65 is moved by the user along the upper surface 34A of the compression plate 34 in a state in which a sheet-shaped acoustic matching member 90 (see, for example, Figs. 6A and 6B) is provided on an ultrasonic wave transmitting and receiving surfaces by an attachment 80 (see, for example, Figs. 6A and 6B).

Figs. 4A and 4B are diagrams illustrating an example of the outward appearance of the ultrasound probe 65 without the attachment 80 in this embodiment. Fig. 4A is a front view illustrating an example of the outward appearance of the ultrasound probe 65 and Fig. 4B is a side view illustrating an example of the outward appearance of the ultrasound probe 65.

As illustrated in Figs. 4A and 4B, a housing 65D of the ultrasound probe 65 according to this embodiment includes a grip portion 65A that is gripped by the user and a transmitting and receiving unit 65B having a transmitting and receiving surface 65C for transmitting and receiving ultrasonic waves.

The ultrasound probe 65D comprises a plurality of ultrasound transducers (not illustrated) which are one-dimensionally or two-dimensionally arranged along the transmitting and receiving surface 65C. Each of the ultrasound transducers transmits ultrasonic waves on the basis of an applied driving signal, receives ultrasound echoes, and outputs a received signal.

For example, each of the plurality of ultrasound transducers is a transducer configured by forming electrodes at both ends of a piezoelectric material (piezoelectric body), such as a piezoelectric ceramic typified by lead (Pb) zirconate titanate (PZT) or a polymeric piezoelectric element typified by polyvinylidene difluoride (PVDF). In a case in which a pulsed or continuous wave drive signal is transmitted to apply a voltage to the electrodes of the transducer, the piezoelectric body is expanded and contracted. Pulsed or continuous ultrasonic waves are generated from each transducer by the expansion and contraction and the ultrasonic waves are combined to form an ultrasound beam. Each transducer receives the propagated ultrasonic waves and is then expanded and contracted to generate an electric signal. The electric signal is output as an ultrasound received signal and is input to the main body (not illustrated) of the ultrasonography apparatus 16 through a cable 67.

As illustrated in Fig. 4A, two concave portions 70 that are recessed into the housing 65D are provided in a surface of the transmitting and receiving unit 65B of the housing 65D. Further, as illustrated in Fig. 4B, a convex portion 72 that protrudes outward from the housing 65D is provided on a side surface of the transmission and receiving unit 65B of the housing 65D. The convex portion 72 according to this embodiment is provided only on one side surface of the housing 65D. For example, the concave portions 70 and the convex portion 72 according to this embodiment are provided to make it easy for the user to recognize the direction (front and back) of the ultrasound probe 65 and the size and shape thereof are not limited.

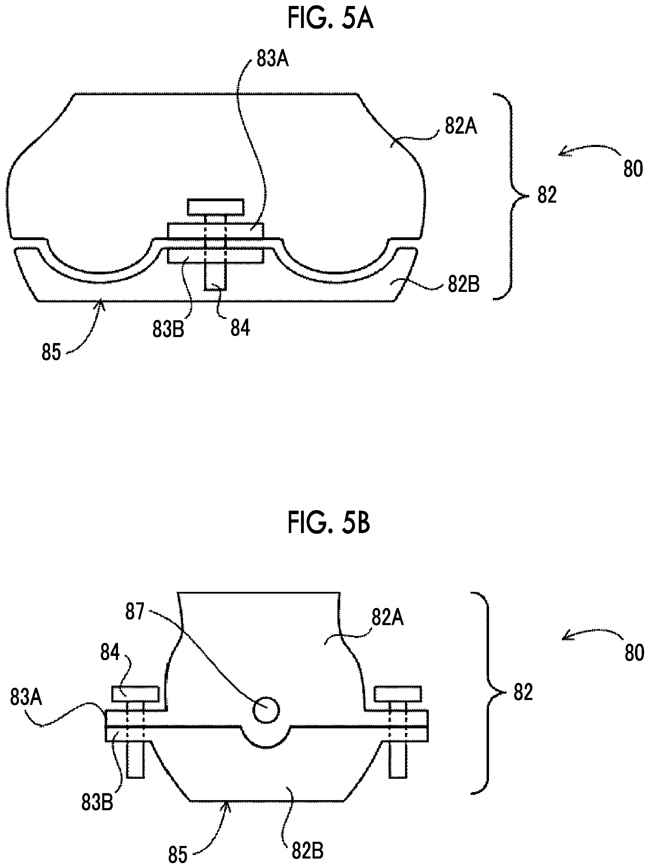

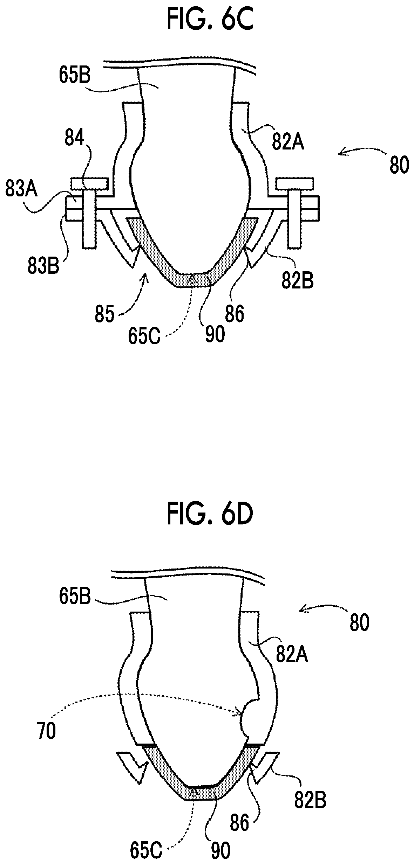

Figs. 5A and 5B are diagrams illustrating an example of the outward appearance of the attachment 80 according to this embodiment. Fig. 5A is a front view illustrating an example of the outward appearance of the attachment 80 and Fig. 5B is a side view illustrating an example of the outward appearance of the attachment 80. Figs. 6A and 6B are diagrams illustrating an example of the outward appearance of the ultrasound probe 65 in a state in which the attachment 80 according to this embodiment is attached. Fig. 6A is a front view illustrating an example of the outward appearance of the ultrasound probe 65 in a state in which the attachment 80 is attached and Fig. 6B is a side view illustrating the outward appearance of the ultrasound probe 65 in a state in which the attachment 80 is attached. Further, Fig. 6C illustrates an example of a cross-sectional view taken along line A-A of Fig. 6A and Fig. 6B illustrates an example of a cross-sectional view taken along line B-B of Fig. 6A.

The attachment 80 according to this embodiment comprises a holder 82. The holder 82 comprises a first holder 82A and a second holder 82B. As illustrated in Figs. 6A and 6B, the first holder 82A is attachably and detachably mounted on the transmitting and receiving unit 65B of the ultrasound probe 65 and is fixed to the ultrasound probe 65. For example, the first holder 82A according to this embodiment is made of a member requiring elasticity such as silicon rubber. Further, the first holder 82A is provided with first fastening portions 83A for attaching the second holder 82B. For example, in this embodiment, the first fastening portions 83A are provided at positions corresponding to the front side and the back side of the ultrasound probe 65, respectively.

As illustrated in Figs. 5B and 6B, an opening portion 87 that fits to the convex portion 72 of the housing 65D of the ultrasound probe 65 is provided in a side surface of the first holder 82A according to this embodiment. The convex portion 72 of the housing 65D of the ultrasound probe 65 is fitted to the opening portion 87 to attach the first holder 82A to the ultrasound probe 65 at an appropriate position and to increase the fixation strength of the attachment 80 to the ultrasound probe 65.

Further, as illustrated in Fig. 6D, the first holder 82A according to this embodiment has a shape that is fitted to the concave portion 70 of the ultrasound probe 65. Therefore, the shape of the first holder 82A is fitted to the concave portion 70 to attach the first holder 82A to the ultrasound probe 65 at an appropriate position and to increase the fixation strength of the attachment 80 to the ultrasound probe 65.

The second holder 82B has an opening portion 85 and is provided with a second fastening portion 83B for attachment to the first holder 82A. For example, in this embodiment, the second fastening portions 83B are provided at positions corresponding to the first fastening portions 83A on the front side and the back side of the ultrasound probe 65, respectively. The first fastening portion 83A and the second fastening portion 83B are fastened with a screw 84 to attach the second holder 82B to the first holder 82A. The screw 84 according to this embodiment is an example of a fastening member according to the present disclosure.

As illustrated in Figs. 6C and 6D, in the second holder 82B, a protruding portion 86 is provided in a tip portion of a surface that faces the ultrasound probe 65. As illustrated in Figs. 6A to 6D, the sheet-shaped acoustic matching member 90 is provided in the opening portion 85 of the second holder 82B. The acoustic matching member 90 is supported by the protruding portion 86 of the second holder 82B. The tip of the protruding portion 86 according to this embodiment is hooked or dug into the acoustic matching member 90 so that the protruding portion 86 draws and holds the acoustic matching member 90 in a direction in which the acoustic matching member 90 is brought into close contact with the transmitting and receiving surface 65C of the ultrasound probe 65. In addition, the protruding portion 86 according to this embodiment presses the acoustic matching member 90 against the ultrasound probe 65 to compress the acoustic matching member 90 against the ultrasound probe 65.

In addition, the acoustic matching member 90 according to this embodiment has a sheet shape and is made of a material that has both high compatibility with the subject (the breast in this embodiment) and high ultrasonic wave transmittance. The acoustic matching member 90 may be, for example, a sheet-shaped solid material or may be a member that is formed by covering a surface of a gel-like material with, for example, silicon rubber and is maintained in a sheet shape.

In a case in which the attachment 80 is attached to the ultrasound probe 65, first, the first holder 82A is attached to the ultrasound probe 65.

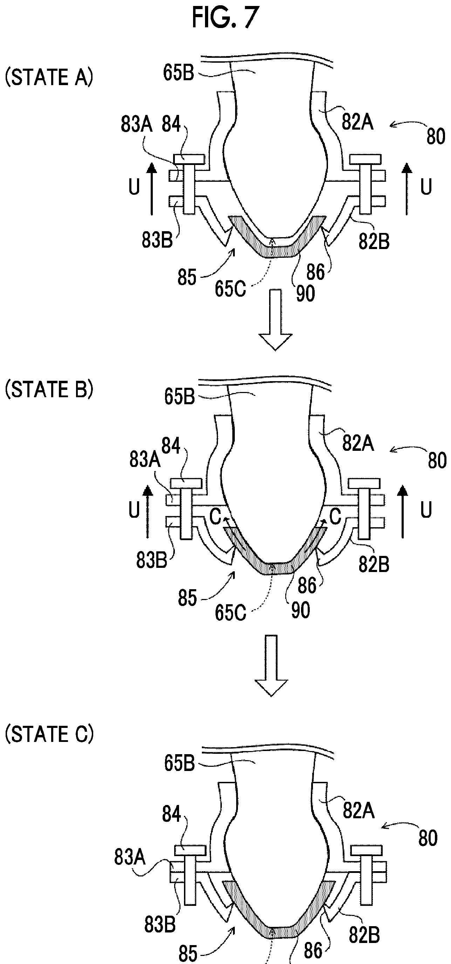

Then, in a state in which the acoustic matching member 90 is provided inside the opening portion 85 of the second holder 82B, the first fastening portion 83A of the first holder 82A and the second fastening portion 83B of the second holder 82B are gradually fastened with the screw 84. The acoustic matching member 90 is brought into close contact with the transmitting and receiving surface 65C of the ultrasound probe 65 by the attachment of the second holder 82B to the first holder 82A. A mechanism in which the acoustic matching member 90 is brought into close contact with the transmitting and receiving surface 65C of the ultrasound probe 65 by the attachment 80 will be described with reference to Fig. 7. Fig. 7 is a cross-sectional view that corresponds to Fig. 6C and illustrates the attachment 80 and the acoustic matching member 90.

As illustrated in Fig. 7, the first holder 82A and the second holder 82B are fastened with the screw 84 to draw the second holder 82B in the direction of an arrow U. In the example illustrated in a state A of Fig. 7, the second holder 82B is drawn in the direction of the arrow U. As illustrated in the state A of Fig. 7, in a case in which the screw 84 is loosely fastened and the first holder 82A and the second holder 82B are relatively separated from each other, a gap between the acoustic matching member 90 and the transmitting and receiving surface 65C of the ultrasound probe 65 is likely to be formed.

In a case in which the amount of tightening of the screw 84 is increased, the second holder 82B is moved in the direction of the arrow U as illustrated in a state B of Fig. 7 and the acoustic matching member 90 and the transmitting and receiving surface 65C of the ultrasound probe 65 come into contact with each other.

Further, in a case in which the amount of tightening of the screw 84 is increased, the protruding portion 86 of the second holder 82B draws the acoustic matching member 90 in a close contact direction (see an arrow C in Fig. 7) in which the acoustic matching member 90 is brought into close contact with the transmitting and receiving unit 65B in a state in which the acoustic matching member 90 is brought into close contact with the transmitting and receiving surface 65C. Therefore, as illustrated in a state C of Fig. 7, in a state in which the screw 84 is tightened to completely attach the second holder 82B to the first holder 82A, the protruding portion 86 holds the acoustic matching member 90 while bringing the acoustic matching member 90 into close contact with the transmitting and receiving surface 65C. In this embodiment, in the process from the state B to the state C, in a state in which the acoustic matching member 90 is brought into close contact with the transmitting and receiving surface 65C, the protruding portion 86 draws the acoustic matching member 90 in the close contact direction in which the acoustic matching member 90 is brought into close contact with the transmitting and receiving unit 65B to push out a foreign material, such as air, between the acoustic matching member 90 and the transmitting and receiving surface 65C. Therefore, the acoustic matching member 90 and the transmitting and receiving surface 65C can be brought into closer contact with each other and it is possible to obtain a high-quality ultrasound image.

As illustrated in the state C of Fig. 7, in a state in which the attachment of the second holder 82B to the first holder 82A is completed, the acoustic matching member 90 protrudes from the tip (opening end) of the opening portion of the second holder 82B. The tip of the transmitting and receiving surface 65C of the ultrasound probe 65 also protrudes from the opening end of the second holder 82B.

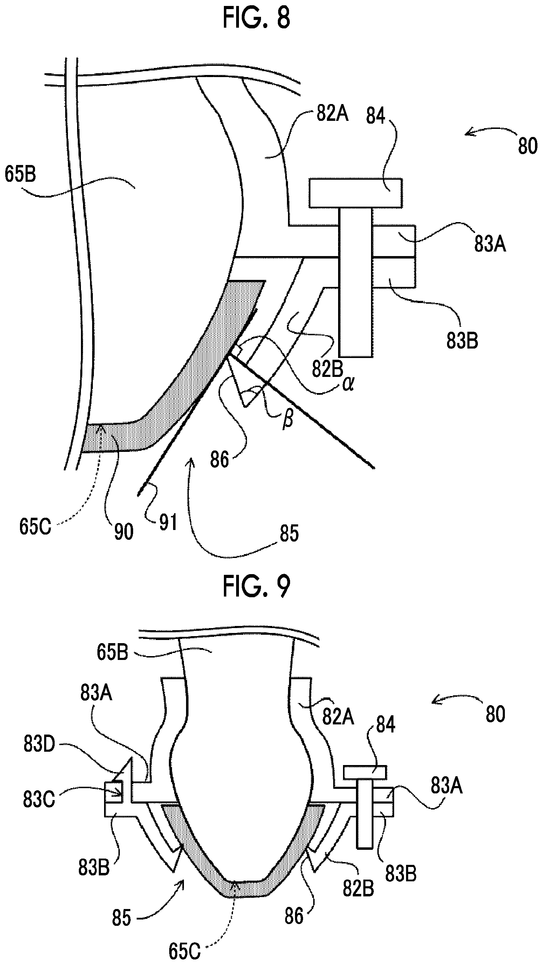

As described above, in order for the tip of the protruding portion 86 to draw the acoustic matching member 90 in the close contact direction in which the acoustic matching member 90 is brought into close contact with the transmitting and receiving section 65B, an angle α between the tip of the protruding portion 86 and a surface 91 of the acoustic matching member 90 with which the tip of the protruding portion 86 comes into contacts is preferably equal to or greater than 70 degrees and equal to or less than 120 degrees and more preferably about 90 degrees, as illustrated in Fig. 8. In this case, an angle β of the protruding portion 86 is preferably equal to or greater than 30 degrees and equal to or less than 45 degrees.

In addition, in the attachment 80 according to this embodiment, the amount of tightening of the screw 84 may be variable. Therefore, the attachment 80 can also correspond to various acoustic matching members 90 having different thicknesses.

The structure for fastening the first holder 82A and the second holder 82B is not limited to the above-described structure. For example, a structure according to the following Modification Example 1 may be applied.

### Modification Example 1

Fig. 9 is a cross-sectional view illustrating an example of the holder 82 and the acoustic matching member 90 in a state in which an attachment 80 according to this modification is attached. Fig. 9 illustrates an example of a structure in which the first holder 82A and the second holder 82B are fastened with a so-called snap-fit, instead of fastening the first holder 82A and the second holder 82B with the screw 84.

As illustrated in Fig. 9, an opening portion 83C is provided in the first fastening portion 83A of the first holder 82A. The second fastening portion 83B of the second holder 82B is provided with a cantilever 83D. In the example illustrated in Fig. 9, the cantilever 83D of the second holder 82B is inserted into the opening portion 83C of the first holder 82A and is then hooked to the first fastening portion 83A. In this way, the second holder 82B is fastened to the first holder 82A. The opening portion 83C and the cantilever 83D according to this modification example are examples of a fastening member according to the present disclosure.

In the example illustrated in Fig. 9, one of the front side and the back side of the ultrasound probe 65 is fastened to the first holder 82A and the second holder 82B with the snap-fit. However, both the front side and the back side of the ultrasound probe 65 may be fastened to the first holder 82A and the second holder 82B with the snap-fits.

In a case in which the first holder 82A and the second holder 82B are fastened by the snap-fit (cantilever 83D), a portion protruding from the surface of the ultrasound probe 65 tends to be smaller than that in a case in which the first holder 82A and the second holder 82B are fastened with the screw 84. In a case in which an ultrasound image of the breast compressed by the compression plate 34 is captured in the mammography apparatus 10 as in the ultrasonography apparatus 16 using the ultrasound probe 65 according to this embodiment, it is desirable to capture an ultrasound image of a part closer to the chest wall of the breast of the subject. In a case in which there is a protruding portion on the surface of the ultrasound probe 65, the protruding portion may be an obstacle, which makes it difficult for the ultrasound probe 65 to approach the chest wall. As in the attachment 80 according to this modification example illustrated in Fig. 9, the snap-fit structure formed by the opening portion 83C and the cantilever 83D makes it possible to reduce the size of the portion protruding from the surface of the ultrasound probe 65 and makes it easy to scanning a part closer to the chest wall. Therefore, it is possible to capture an ultrasound image in a wider range.

The second holder 82B illustrated in, for example, Figs. 5B, 6B, and 6C has a shape along the transmitting and receiving surface 65C that is a portion of the ultrasound probe 65 to which the second holder 82B is attached. However, the structure of the second holder 82B is not limited to this embodiment. For example, structures according to the following Modification Examples 2 and 3 may be applied.

### Modification Example 2

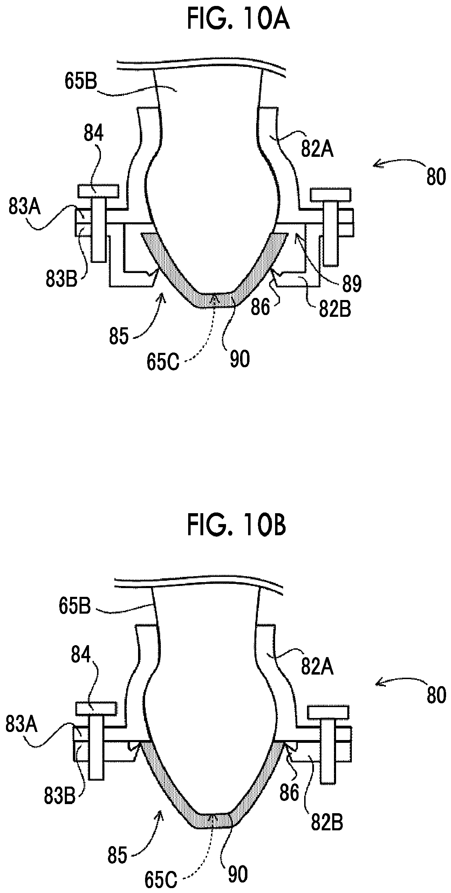

Fig. 10A is a cross-sectional view illustrating an example of a holder 82 and an acoustic matching member 90 in a state in which an attachment 80 according to this modification example is attached. A second holder 82B illustrated in Fig. 10A has an L-shape in a cross-section view and a protruding portion 86 is provided at the tip of the second holder 82B.

In the example illustrated in Fig. 10A, a gap 89 between the first holder 82A and the second holder 82B in a case in which the second holder 82B is fastened to the first holder 82A is larger than that in, for example, Fig. 6C. The gap 89 is increased such that a margin can be given to the length of the acoustic matching member 90 to be disposed.

### Modification Example 3

Fig. 10B is a cross-sectional view illustrating an example of a holder 82 and an acoustic matching member 90 in a state in which an attachment 80 according to this modification example is attached. A second holder 82B illustrated in Fig. 10B has a plate shape and a protruding portion 86 is provided at the tip of the second holder 82B.

In the example illustrated in Fig. 10B, an opening portion 85 can be configured to be larger than that in the second holder 82B illustrated in, for example, Figs. 6C and 10A. In the example illustrated in Fig. 10B, it is possible to simplify the second holder 82B.

### Second Embodiment

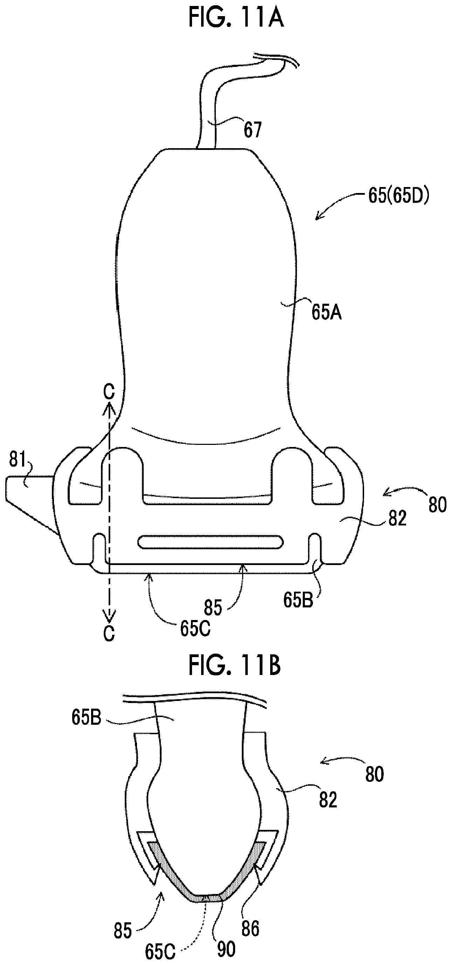

Next, a second embodiment will be described in detail. Fig. 11A is a diagram illustrating an example of the outward appearance of an ultrasound probe 65 in a state in which an attachment 80 according to this embodiment is attached. Fig. 11B illustrates an example of a cross-sectional view taken along line C-C of Fig. 11A.

As illustrated in Figs. 11A and 11B, the attachment 80 according to this embodiment comprises a holder 82 having an opening portion 85 and a protruding portion 86 that is provided at the tip (opening end) of the opening portion 85. That is, the holder 82 of the attachment 80 according to this embodiment can be regarded as having a shape in which the first holder 82A and the second holder 82B of the holder 82 according to the first embodiment are integrated.

The attachment 80 according to this embodiment is attached to the ultrasound probe 65 from the side of the transmitting and receiving surface 65C in a state in which the acoustic matching member 90 is provided in the opening portion 85. In a case in which the attachment 80 is attached in this way, the protruding portion 86 draws the acoustic matching member 90 in the close contact direction in which the acoustic matching member 90 is brought into close contact with the transmitting and receiving unit 65B in a state in which the acoustic matching member 90 is brought into close contact with the transmitting and receiving surface 65C, similarly to the attachment 80 according to the first embodiment. Therefore, in the attachment 80 according to this embodiment, it is possible to push out a foreign material, such as air, between the acoustic matching member 90 and the transmitting and receiving surface 65C. Therefore, the acoustic matching member 90 and the transmitting and receiving surface 65C can be brought into closer contact with each other and it is possible to obtain a high-quality ultrasound image.

Further, according to the attachment 80 of this embodiment, since the attachment 80 is formed of one member, it is easy to attach the attachment to the ultrasound probe 65.

Further, a handle portion 81 is provided on one side surface of the holder 82 according to this embodiment. In a case in which the attachment 80 is removed from the ultrasound probe 65 having the attachment 80 according to this embodiment attached thereto, the user can easily attach and detach the attachment 80 by putting a finger on the handle portion 81 and applying force in the direction of the transmitting and receiving surface 65C (downward direction in Fig. 11A).

### Third Embodiment

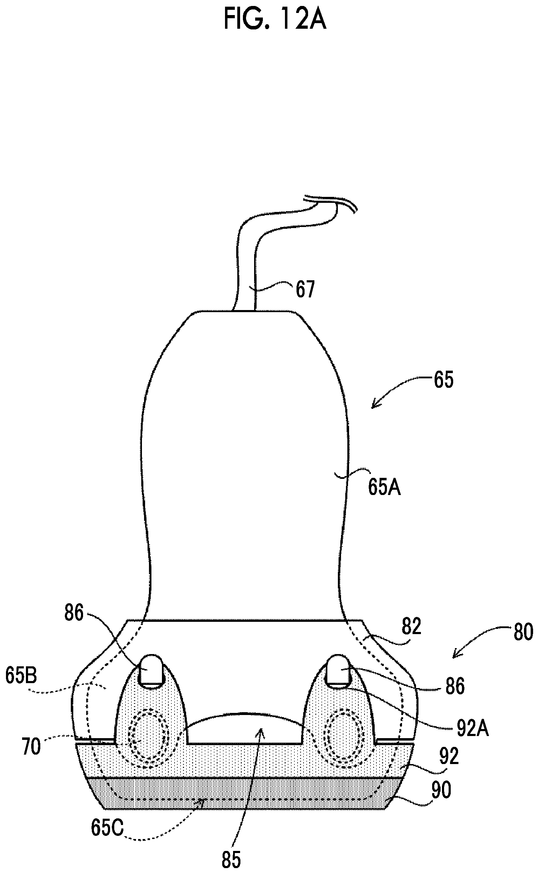

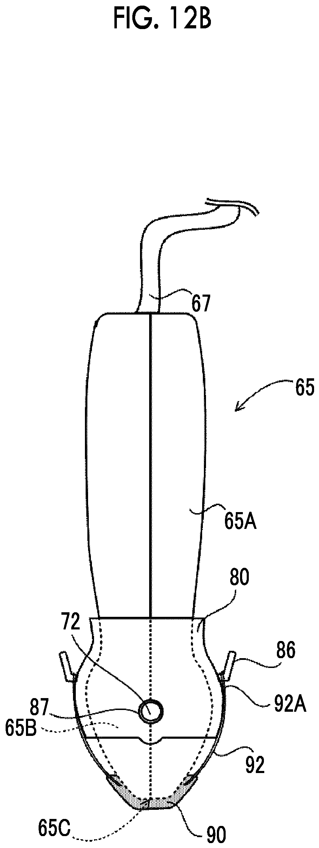

Next, a third embodiment will be described in detail. Figs. 12A and 12B are diagrams illustrating an example of the outward appearance of the ultrasound probe 65 in a state in which am attachment 80 according to this embodiment is attached. Fig. 12A is a front view illustrating an example of the outward appearance of the ultrasound probe 65 having the attachment 80 attached thereto and Fig. 12B is a side view illustrating an example of the outward appearance of the ultrasound probe 65 having the attachment 80 attached thereto.

As illustrated in Figs. 12A and 12B, the attachment 80 according to this embodiment comprises a holder 82 having an opening portion 85. The holder 82 is detachably attached to the transmitting and receiving unit 65B of the ultrasound probe 65 and is fixed to the ultrasound probe 65. For example, the holder 82 according to this embodiment is made of a member requiring elasticity such as silicon rubber. In addition, the holder 82 has protruding portions 86 provided on a surface which is opposite to a surface facing the ultrasound probe 65. For example, in this embodiment, two protruding portions 86 are provided on each of the front side and the back side of the ultrasound probe 65. The protruding portion 86 is a so-called hook that protrudes outward from the ultrasound probe 65.

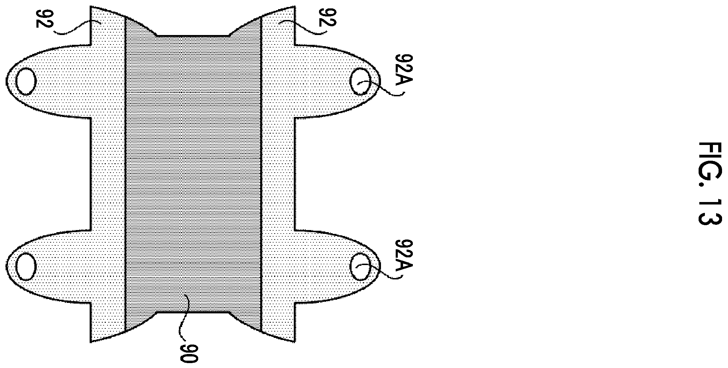

In contrast, an acoustic matching member 90 according to this embodiment is provided with an attachment member 92. Fig. 13 is a plan view illustrating an example of the acoustic matching member 90 according to this embodiment. As illustrated in Fig. 13, in the acoustic matching member 90 according to this embodiment, the attachment member 92 is provided on each of opposes sides. The attachment member 92 is a member that has elasticity and is made of, for example, nonwoven fabric or silicon rubber. A method for providing the attachment member 92 in the acoustic matching member 90 is not particularly limited. For example, the attachment member 92 may be provided in the acoustic matching member 90 by burying the end of the attachment member 92 in the acoustic matching member 90. Further, the acoustic matching member 90 and the attachment member 92 may be attachable and detachable.

Holes 92A are provided on a side of the attachment member 92, which is opposite to the side on which the attachment member 92 is provided in the acoustic matching member 90, at positions corresponding to the protruding portions 86 of the holder 82. In this embodiment, as described above, since two protruding portions 86 of the holder 82 are provided on each of the front side and the back side of the ultrasound probe 65, two holes 92A are provided on each side of the attachment member 92. In a case in which the attachment member 92 is attached by fitting the hole 92A to each of the protruding portions 86, the protruding portions 86 draws the acoustic matching member 90 in the close contact direction in which the acoustic matching member 90 is brought into close contact with the transmitting and receiving surface 65C of the ultrasound probe 65.

In a case in which the attachment 80 according to this embodiment is attached to the ultrasound probe 65, first, the holder 82 is attached to the attachment 80. Then, in a state in which the acoustic matching member 90 is brought into close contact with the transmitting and receiving surface 65C of the ultrasound probe 65, the attachment member 92 is drawn in the close contact direction in which the acoustic matching member 90 is brought into close contact with the transmitting and receiving surface 65C of the ultrasound probe 65 and each of the holes 92A is fitted to the protruding portion 86. In a case in which the attachment member 92 is drawn in the close contact direction, the attachment member 92 is pressed by hand in the close contact direction from the transmitting and receiving surface 65C to push out a foreign material, such as air, between the transmitting and receiving surface 65C (transmitting and receiving unit 65B) of the ultrasound probe 65 and the acoustic matching member 90. Therefore, the acoustic matching member 90 and the transmitting and receiving surface 65C can be brought into closer contact with each other and it is possible to obtain a high-quality ultrasound image.

As described above, the attachment 80 according to each of the above-described embodiments is provided with the opening portion 85 that provided at a position corresponding to the ultrasonic wave transmitting and receiving surface 65C of the ultrasound probe 65 and comprises the holder 82 that is attachably and detachably fixed to the ultrasound probe 65 and the protruding portions 86 that draw and hold the sheet-shaped acoustic matching member 90 provided between the opening portion 85 and the ultrasound probe 65 in the close contact direction in which the acoustic matching member 90 is brought into close contact with the transmitting and receiving surface 65C.

In the attachment 80 according to each of the above-described embodiments, the protruding portion 86 draws and holds the sheet-shaped acoustic matching member 90 in the close contact direction in which the acoustic matching member 90 is brought into close contact with the transmitting and receiving surface 65C of the ultrasound probe 65. Therefore, it is not necessary to apply or wipe the acoustic matching member 90 on the surface scanned by the ultrasound probe 65.

Therefore, it is possible to further improve the convenience of the user who takes an ultrasound image with the ultrasonography apparatus 16 using the ultrasound probe 65.

Further, in the medical imaging system 1 according to each of the above-described embodiments, in a case in which an ultrasound image of the breast (subject) is captured, the compression plate 34 of the mammography apparatus 10 is moved in the compression direction and imaging is performed in a state in which the breast is compressed. In many cases, while the breast is compressed by the compression plate 34, the capture of a radiographic image by the radiation detector 30 and the capture of an ultrasound image by the ultrasonography apparatus 16 are continuously performed. In this case, the breast is continuously compressed by the compression plate 34 before the capture of a radiographic image is started and until the capture of an ultrasound image is completed. For example, in a case in which an acoustic matching member other than the acoustic matching member according to this embodiment is applied, for example, in a case in which a gel-like acoustic matching member is used, the acoustic matching member is applied onto and removed from the upper surface 34A of the compression plate 34 compressing the breast.

In contrast, in a case in which an ultrasound image is captured using the ultrasound probe 65 provided with the attachment 80 according to each of the above-described embodiments, it is not necessary to apply and remove the acoustic matching member onto and from the upper surface 34A of the compression plate 34. Therefore, it is possible to reduce the time for which the breast is compressed by the compression plate 34. Therefore, the use of the ultrasound probe 65 provided with the attachment 80 according to this embodiment makes it possible to reduce a burden on the subject.

Further, according to the attachment 80 of each of the above-described embodiments, it is possible to prevent a foreign material, such as air, from entering between the transmitting and receiving surface 65C of the ultrasound probe 65 and the acoustic matching member 90. Therefore, according to the attachment 80 of each of the above-described embodiments, the acoustic matching member 90 and the transmitting and receiving surface 65C can be brought into closer contact with each other and it is possible to obtain a high-quality ultrasound image.

In addition, according to the attachment 80 of each of the above-described embodiments, the attachment 80 enables the user to easily scan the subject with the ultrasound probe 65 in a state in which the angle of the ultrasound probe 65 with respect to the subject is closer to a right angle (90 degrees). Therefore, it is possible to obtain a high-quality ultrasound image.

In each of the above-described embodiments, the aspect in which the attachment 80 is attached to the ultrasound probe 65 scanning the upper surface 34A of the compression plate 34 of the mammography apparatus 10 has been described. However, the present disclosure is not limited thereto. The attachment 80 according to each of the above-described embodiments may be attached to other ultrasound probes 65. For example, in a state in which the ultrasound probe 65 having the attachment 80 attached thereto is brought into contact with the subject, specifically, in a state in which the acoustic matching member 90 provided in the attachment 80 is brought into contact with the subject, scanning is performed with the ultrasound probe 65 to capture an ultrasound image of the subject.

For example, the configurations and operations of the medical imaging system 1, the mammography apparatus 10, the ultrasonography apparatus 16, the ultrasound probe 65, and the attachment 80 described in each of the above-described embodiments are illustrative and may be changed according to the situation, without departing from the scope and spirit of the invention. In addition, the above-described embodiments may be appropriately combined with each other.

### Explanation of References

1: medical imaging system
2: radiography system
5: RIS
10: mammography apparatus
12: console
16: ultrasonography apparatus
18: image storage system
20, 60: control unit
22, 62: storage unit
24, 64: I/F unit
30: radiation detector
32: compression plate driving unit
34: compression plate
34A: upper surface
36: radiation emitting unit
36R: radiation source
40: imaging table
40A: imaging surface
42: arm portion
44: base
45: shaft portion
46: compression unit
60A: CPU
60B: ROM
60C: RAM
65: ultrasound probe
65A: grip portion
65B: transmitting and receiving unit
65C: transmitting and receiving surface
65D: housing
66: operation unit
67: cable
68: display unit
69: bus
70: concave portion
72: convex portion
80: attachment
81: handle portion
82: holder
82A: first holder
82B: second holder
83A: first fastening portion
83B: second fastening portion
83C: opening portion
85D: cantilever
84: screw
85, 87: opening portion
86: protruding portion
89: gap
90: acoustic matching member
91: surface
92: attachment member
92A: hole
C, U: arrow
R: radiation
α, β: angle

## Claims

1. An attachment for an ultrasound probe comprising:
a holder that has an opening portion provided at a position corresponding to an ultrasonic wave transmitting and receiving surface of the ultrasound probe and is attachably and detachably fixed to the ultrasound probe; and
a protruding portion that draws and holds a sheet-shaped acoustic matching member provided between the opening portion and the ultrasound probe in a close contact direction in which the acoustic matching member is brought into close contact with the transmitting and receiving surface.

2. The attachment according to claim 1,
wherein the protruding portion is provided on a surface of the holder which faces the ultrasound probe.

3. The attachment according to claim 1,
wherein the holder has a first holder that is attachably and detachably fixed to the ultrasound probe and a second holder that is attached to the first holder and has the opening portion, and
the protruding portion is provided on a surface of the second holder which faces the ultrasound probe.

4. The attachment according to claim 3,
wherein the second holder is attached to the first holder by being fastened to the first holder with a fastening member.

5. The attachment according to claim 4,
wherein the protruding portion draws the acoustic matching member in the close contact direction by fastening the second holder to the first holder with the fastening member.

6. The attachment according to any one of claims 2 to 5,
wherein an angle formed between a tip of the protruding portion and a contact surface of the acoustic matching member with the tip is equal to or greater than 70 degrees and equal to or less than 120 degrees.

7. The attachment according to any one of claims 1 to 6,
wherein the acoustic matching member protrudes from an opening end of the holder in a state in which the attachment is attached to the ultrasound probe.

8. The attachment according to any one of claims 1 to 7,
wherein a tip of the transmitting and receiving surface of the ultrasound probe protrudes from the opening end of the holder in a state in which the attachment is attached to the ultrasound probe.

9. The attachment according to claim 1,
wherein the acoustic matching member includes an elastic attachment member which is provided on each of a pair of opposite sides,
the protruding portion is provided on a surface of the holder which is opposite to a surface facing the ultrasound probe, and
the attachment member is attached to the protruding portion to draw the acoustic matching member in the close contact direction.

10. The attachment according to any one of claims 1 to 9,
wherein a surface of the ultrasound probe has at least one of a convex portion or a concave portion, and
the holder has a shape that is fitted to the at least one.

11. An ultrasound probe to which the attachment according to any one of claims 1 to 10 is attached.
